# EUROPÄISCHE PATENTANMELDUNG

(11) **EP 4 834 233 A1**
(43) Veröffentlichungstag der Anmeldung: **04.11.2026**
(21) Anmeldenummer: 25173401.8
(22) Anmeldetag: 29.04.2025
(51) Int. Cl.: A61B 6/10

(54) **STRAHLENSCHUTZVORRICHTUNG**

(71) Anmelder: MAVIG GmbH, 81829 München (DE)
(72) Erfinder: SIMMONDS, Ryan, 2244BL Wassenaar (NL)
(74) Vertreter: Vossius & Partner Patentanwälte Rechtsanwälte mbB

(57) **Zusammenfassung**

Es ist eine Strahlenschutzvorrichtung (10) offenbart, aufweisend: ein erstes Strahlenschutzpaneel (12), ein von dem ersten Strahlenschutzpaneel (12) separat angeordnetes zweites Strahlenschutzpaneel (14), wobei die beiden Strahlenschutzpaneele (12, 14) derart zueinander angeordnet sind, dass zwischen den beiden Strahlenschutzpaneelen (12, 14) ein für Strahlung durchlässiger Spalt (24) vorhanden ist oder sich im Laufe der Prozedur durch Veränderungen der Positionen der beiden Strahlenschutzpaneele (12, 14) ergibt, und ein an dem ersten Strahlenschutzpaneel (12) und an dem zweiten Strahlschutzpaneel (14) befestigbares und den Spalt (24) schließendes Strahlenschutzverbindungselement (26), wobei das Strahlenschutzverbindungselement (26) derart flexibel ausgebildet ist, dass der Spalt (24) bei Bewegung und/oder bei einer unterschiedlichen Positionierung der Strahlenschutzpaneele (12, 14) geschlossen bleibt.

## Beschreibung

### Technisches Gebiet der Erfindung

Die vorliegende Erfindung betrifft eine Strahlenschutzvorrichtung. Insbesondere betrifft die Erfindung eine Strahlenschutzvorrichtung, die beispielsweise zur Verwendung an einem Arbeitsplatz der interventionellen Radiologie geeignet ist und die zum Schutz von Personen wie einem Arzt oder Assistenten gegen Strahlung, insbesondere gegen Röntgenstrahlung, dient.

### Hintergrund

Strahlenschutzvorrichtungen dienen dem Schutz von Personen vor schädlicher energiereicher Strahlung, beispielsweise Röntgenstrahlung.

Bekannte Strahlenschutzlösungen umfassen beispielsweise sogenannte Strahlenschutzscheiben. Diese sind starr, meist an einem Tragarm befestigt und in ihrer Position variabel einstellbar. Andere bekannte Strahlenschutzlösungen umfassen mobile Strahlenschutzwände, die z.B. verfahrbar sind.

Unter bestimmten Umständen kann es vorkommen, dass die Verwendung einer einzigen Strahlenschutzwand oder Strahlenschutzscheibe nicht ausreichend ist, beispielsweise wenn Personen auf beiden Seiten eines Untersuchungstischs arbeiten müssen.

Aufgabe der vorliegenden Erfindung ist es daher, eine Strahlenschutzvorrichtung bereitzustellen, die einen gegenüber einer einzelnen Strahlenschutzwand oder Strahlenschutzscheibe vergrößerten Schutzbereich bietet und zudem multifunktional nutzbar und flexibel einsetzbar ist.

### Lösung des Problems

Diese und andere Aufgaben werden durch den Gegenstand des unabhängigen Anspruchs 1 gelöst. Bevorzugte Ausgestaltungen sind in den abhängigen Ansprüchen angegeben.

Gemäß eines Aspekts der vorliegenden Erfindung wird eine Strahlenschutzvorrichtung angegeben. Die Strahlenschutzvorrichtung weist auf: ein erstes Strahlenschutzpaneel, ein von dem ersten Strahlenschutzpaneel separat angeordnetes zweites Strahlenschutzpaneel, wobei die beiden Strahlenschutzpaneele derart zueinander angeordnet sind, dass zwischen den beiden Strahlenschutzpaneelen ein für Strahlung, beispielsweise Röntgenstrahlung, durchlässiger Spalt vorhanden ist oder sich im Laufe der Prozedur durch Veränderungen der Positionen der beiden Strahlenschutzpaneele ergeben kann, und ein an dem ersten Strahlenschutzpaneel und an dem zweiten Strahlschutzpaneel befestigbares und den Spalt schließendes Strahlenschutzverbindungselement, wobei das Strahlenschutzverbindungselement derart flexibel ausgebildet ist, dass der Spalt bei Bewegung und/oder unterschiedlicher Positionierung der beiden Strahlenschutzpaneele geschlossen bleibt.

Die im Rahmen dieser Offenbarung bezeichneten Strahlenschutzpaneele können auch als Strahlenschutzplatten oder Strahlenschutzscheiben oder Obertisch-Streustrahlenschutz bezeichnet werden, wobei diese sowohl transparent als auch nicht-transparent sein können. Die Strahlenschutzpaneele sind üblicherweise relativ steif bzw. starr und nicht und nur minimal flexibel oder biegsam. Die Strahlenschutzpaneele können an Tragarmen befestigt und in unterschiedlichen Positionen angeordnet werden. Die Strahlenschutzpaneele können aber auch mobile Strahlenschutzwände sein, die verfahrbar sind.

Die vorliegende Erfindung geht von der Erkenntnis aus, dass bei Verwendung mehrerer solcher steifer bzw. starrer Strahlenschutzpaneele ein Spalt zwischen den Paneelen existieren kann. Dieser Spalt weist kein strahlenabsorbierendes Material auf und ist daher prinzipiell für schädliche Strahlung, wie beispielsweise Streustrahlung einer Strahlungsquelle (Röntgenstrahlungsquelle) und/oder Sekundärstrahlung, welche beispielsweise durch den Patentientenkörper in Richtung zum Personal (beispielsweise Arzt oder Assistenz) austritt, durchlässig. Die Erfindung beruht auf der Idee, diesen Spalt mit einem flexiblen Strahlenschutzverbindungselement zu schließen. Das Strahlenschutzverbindungselement weist ein strahlenabsorbierendes Material auf, schließt bzw. verdeckt den Spalt und schützt damit Personen, wie beispielsweise das Personal, vor der schädlichen Strahlung. Der Strahlenschutz wird als Barriere so positioniert, dass ein größtmöglicher Bereich des Patientenkörper und/oder des im Raum anwesenden Personals abgeschirmt wird.

Das Strahlenschutzverbindungselement ist flexibel ausgebildet. Mit "flexibel" ist gemeint, dass das Strahlenschutzverbindungselement nicht starr ist, wie die beschriebenen Strahlenschutzpaneele, sondern biegsam und formveränderlich, ähnlich wie eine strahlenabsorbierende Schutzkleidung. Mit anderen Worten kann sich das Strahlenschutzverbindungselement an sich verändernde Positionen der (starren) Strahlenschutzpaneele anpassen, sodass der Spalt auch bei Bewegungen und/oder veränderten Positionen der Strahlenschutzpaneele durch das Strahlenschutzverbindungselement geschossen bleibt.

Die erfindungsgemäße Strahlenschutzvorrichtung ermöglicht es, zwei oder mehr Strahlenschutzpaneele miteinander zu kombinieren, ohne dass Personen durch einen zwischen den Paneelen existierenden Spalt der schädlichen Strahlung ausgesetzt sind. Dadurch können beispielsweise aufwendige Untersuchungen, die Personen auf beiden Seiten eines Untersuchungstischs benötigen, durchgeführt werden, ohne die Personen der schädlichen Strahlung auszusetzen. Die erfindungsgemäße Strahlenschutzvorrichtung erweitert damit die effektiv wirksame Strahlenschutzfläche (Reichweite) einer einzelnen Strahlenschutzscheibe oder mobilen Strahlenschutzwand und stellt eine flexible und multifunktionale Strahlenschutzlösung bereit.

Bevorzugt ist das Strahlenschutzverbindungselement nicht selbsttragend. Mit dem Begriff "nicht selbstragend" ist gemeint, dass das Strahlenschutzverbindungselement ohne Halterung zusammenfallen würde, ähnlich einem (Strahlenschutz)Kleidungsstück. Das Strahlenschutzverbindungselement ist also gerade keine klappbare oder faltbare Strahlenschutzwand oder dergleichen, die - im Gegensatz zum hier beschriebenen Strahlenschutzverbindungselement - selbsttragend wäre. Das Strahlenschutzverbindungselement ist an den Strahlenschutzpaneelen gehalten, wenn es daran befestigt ist.

Bevorzugt ist das Strahlenschutzverbindungselement derart flexibel, dass das Strahlenschutzverbindungselement um eine Kante des ersten und/oder zweiten Strahlenschutzpaneels biegbar ist. Dies ist beispielsweise bei teilweiser überlappender Anordnung der Strahlenschutzpaneele wünschenswert.

Bevorzug sind die Strahlenschutzpaneele in verschiedenen Winkeln zueinander positionierbar und ist das Strahlenschutzverbindungselement dazu ausgebildet, den Spalt in gewinkelten Anordnungen der Strahlenschutzpaneele zu schließen.

Bevorzugt sind die Strahlenschutzpaneele teilweise überlappend zueinander positionierbar und ist das Strahlenschutzverbindungselement dazu ausgebildet, den Spalt bei teilweise überlappender Anordnung der beiden Strahlenschutzpaneele zu schließen. Vorzugweise ist eine Breite des Strahlenschutzverbindungselement derart gewählt, dass ein gewünschter Überlappungsbereich durch das Strahlenschutzverbindungselement abdeckbar ist.

Bevorzugt weist das erste und zweite Strahlenschutzpaneel Befestigungselemente auf, die zum lösbaren Befestigen des Strahlenschutzverbindungselements ausgebildet sind.

Bevorzugt sind die Befestigungselemente in einem Randbereich des jeweiligen Strahlenschutzpaneels angeordnet. Beispielsweise sind bei einem ersten, links angeordneten Strahlenschutzpaneel die Befestigungselemente in einem rechten Randbereich angeordnet und sind bei einem zweiten, rechts angeordneten Strahlenschutzpaneel die Befestigungselemente in einem linken Randbereich angeordnet. Vorzugsweise sind die Befestigungselement von einer Außenkante des jeweiligen Strahlenschutzpaneels derart beabstandet angeordnet, dass das im Randbereich des jeweiligen Paneels befestigte Strahlenschutzverbindungselement zumindest teilweise mit dem jeweiligen Strahlenschutzpaneel überlappt.

Bevorzug weist das Strahlenschutzverbindungselement Gegenelemente auf, die an den Befestigungselementen der Strahlenschutzpaneele befestigbar sind, vorzugsweise wobei die Gegenelemente von den Befestigungselementen zerstörungsfrei und ohne Werkzeug lösbar sind. Das zerstörungsfreie Lösen der Gegenelemente von den Befestigungselementen ermöglicht einen flexiblen Umgang mit den Paneelen. Beispielsweise kann nach dem Lösen der Gegenelemente von den Befestigungselementen eines der Paneele aus dem Arbeitsfeld herausbewegt werden, um beispielsweise nicht zu stören. Wird der Einsatz des herausgeschwenkten Paneels benötigt, wird dieses in das Arbeitsfeld bewegt und werden die Gegenelemente an die Befestigungselemente befestigt. Ein Einsatz von Werkzeug ist nicht notwendig. Die im Arbeitsfeld arbeitenden Personen können das Strahlenschutzverbindungselement eigenständig und ohne Werkzeug an den Paneelen befestigen und von diesen auch wieder lösen. Es ist bevorzugt, dass das Strahlenschutzverbindungselement über Druckknopfverbindungen, Klettverbindungen, Karabiner, Haken und Ösen, Magnete, Saugnäpfe, Klebeverbindung und/oder anderen, zweckmäßigen Verbindungsmitteln lösbar an den Paneelen befestigbar ist.

Es kann jedoch auch eine nicht zerstörungsfreie bzw. eine nicht ohne (geeignetem) Werkzeug lösbare Verbindung zwischen dem Strahlenschutzverbindungselement und den Strahlenschutzpaneelen vorgesehen sein. Beispielsweise könnte es von einem Betreiber einer Röntgeneinrichtung und/oder von einem Strahlenschutzverantwortlichen explizit gewünscht sein, dass das Personal am Arbeitsplatz aus eigenem Antrieb bzw. mit eigenen Mitteln den erweiterten Strahlenschutz bzw. das Strahlenschutzverbindungselement nicht entfernen können darf.

Bevorzugt ist das Strahlenschutzverbindungselement aus einem für Strahlenschutzkleidung geeignetem Material hergestellt und/oder ist das Strahlenschutzverbindungselement aus einem Strahlenschutz-Drape-Material hergestellt. Die Idee ist, für das Strahlenschutzverbindungselement dasselbe oder ein vergleichbares Material wie bei Strahlenschutz-Drapes oder Strahlenschutzkleidung zu verwenden. Diese Materialien haben sich als zuverlässige und flexible Strahlenschutzmaterialien bewährt. Als Beispiele für Materialien können Strahlenschutzmaterialien auf der Basis eines polymeren Compounds (beispielsweise Kautschuk, Silikon, TPU, Vinyl, PVC, Polyethylen, Polyester) mit strahlenabsorbierenden Additiven (beispielsweise Blei, Zinn, Antimon, Bismut, Wolfram, Cer) in unterschiedlichen Zusammensetzungen verwendet werden. Es gibt Normen zu Schutzwerten, Homogenität und Ausführung der Kleidung in Bezug auf Schnittformen und Größen (in der EU z.B. EN 61331-3:2014, in Deutschland DIN EN 61331-3:2016) ebenso wie für die Messmethodik zur Bestimmung der Schwächungseigenschaften (EN 61331-1:2014 bzw. DIN EN 61331-1:2016).

Bevorzugt ist das Strahlenschutzverbindungselement eine Strahlenschutz-Abdeckung (Drape). Die Idee ist, dass das Drape bereits ein geeignetes Strahlenschutzmaterial aufweist. Insofern kann das Drape die Funktion des Strahlenschutzverbindungselements übernehmen und als Strahlenschutzverbindungselement dienen.

Bevorzugt ist das Strahlenschutzmaterial und/oder das Strahlenschutzverbindungselement als Mehrweg- oder Einwegprodukt ausgebildet. Mehrwegprodukte bedürfen nach dem Gebrauch eventuell beispielsweise einer Reinigung, Desinfektion bzw. Sterilisation um den Kautelen der Hygiene im medizinischen Umfeld zu entsprechen. Mehrwegprodukte können über einen längeren Zeitraum zum Einsatz kommen und reduzieren die Abfallmengen. Einwegprodukte dagegen werden nach Gebrauch entsorgt und somit entfällt eine hygienische Aufbereitung. Jedoch werden dabei wertvolle metallische Stoffe (strahlenabsorbierende Additive) nach einmaligem Gebrauch des Strahlenschutzverbindungselements dem Abfall zugeführt. Bei einem als Einwegprodukt ausgebildeten Strahlenschutzverbindungselement kann das Strahlenschutzverbindungselement beispielsweise mittels einer Klebeverbindung oder einer anderen, zweckmäßigen Verbindung an den Strahlenschutzpaneelen anbringbar sein bzw. angebracht sein. Vorzugsweise kann das Strahlenschutzverbindungselement mittels Lösen der Klebeverbindung und/oder der anderen, zweckmäßigen Verbindung von den Strahlenschutzpaneelen gelöst werden, vorzugweise zerstörungsfrei und ohne Einsatz von Werkzeug.

Bevorzug weist Strahlenschutzverbindungselement eine Außenhülle auf und ist in einem Inneren der Außenhülle ein geeignetes Strahlenschutzmaterial angeordnet. Das Strahlenschutzmaterial kann in die Außenhülle eingearbeitet sein, welche aus einem textilen Werkstoff aus Naturfasern oder Chemiefasern oder einem kunststoffbasierenden Werkstoff wie beispielsweise Elastomeren, besteht. Eine Außenhülle schützt das innenliegende Strahlenschutzmaterial und verleiht zusätzliche Stabilität (Robustheit). Das Strahlenschutzverbindungselement kann auch ohne Außenhülle verarbeitet sein. Hierbei besteht die Möglichkeit der Sichtkontrolle hinsichtlich etwaiger Läsionen des Strahlenschutzmaterials, beispielsweise im Rahmen der Qualitätskontrolle der im Gebrauch befindlichen Strahlenschutzausrüstung.

Bevorzugt ist das Strahlenschutzverbindungselement reinigbar und/oder desinfizierbar. Bevorzugt ist das Strahlenschutzverbindungselement sterilisierbar und/oder mit einer sterilen oder sterilisierbaren Umhüllung (Außenhülle) ausgerüstet, falls die jeweilige Prozedur dies aus hygienischer Sicht erfordert.

Bevorzugt weist das Strahlenschutzverbindungselement einen Bleigleichwert Pb in einem Bereich von 0,25 mm bis 1,5 mm, vorzugsweise in einem Bereich von 0,35 mm bis 0, 5 mm, weiter vorzugsweise 0,5 mm oder 1,0 mm oder einen auf den Bleigleichwert der Strahlenschutzpaneele angeglichenen Bleigleichwert, auf.

Bevorzugt weist das Strahlenschutzverbindungselement ein Gewicht in einem Bereich von 0,3 kg bis 10 kg, vorzugsweise in einem Bereich von 1 kg bis 6 kg, weiter vorzugsweise in einem Bereich von 1 kg bis 4 kg, auf, und/oder weist das Strahlenschutzverbindungselement ein Gewicht von maximal 10 kg, vorzugsweise maximal 5 kg, weiter vorzugsweise maximal 4 kg aufweist.

Bevorzugt weist das Strahlenschutzverbindungselement eine Höhe (beziehungsweise Länge) in einem Bereich von 40 cm bis 150 cm, vorzugsweise in einem Bereich von 50 cm bis 120 cm, weiter vorzugsweise in einem Bereich von 60 cm bis 100 cm aufweist, und/oder weist das Strahlenschutzverbindungselement eine Höhe (beziehungsweise Länge) in einem Bereich einer Höhe der Strahlenschutzpaneele auf, und/oder weist das Strahlenschutzverbindungselement eine Breite (senkrecht zur Höhe bzw. Länge) in einem Bereich von 10 cm bis 60 cm, vorzugsweise in einem Bereich von 20 cm bis 50 cm auf, und/oder weist das Strahlenschutzverbindungselement eine im Wesentlichen rechteckige Form aufweist, wobei eine Höhe (beziehungsweise Länge) des Strahlenschutzverbindungselements größer als eine Breite des Strahlenschutzverbindungselements ist, vorzugsweise wobei die Höhe um einen Faktor in einem Bereich von 1 bis 20, vorzugweise in einem Bereich von 1 bis 10, weiter vorzugsweise in einem Bereich von 1 bis 5, größer als die Breite ist.

Bevorzugt weist das Strahlenschutzverbindungselement ein oder mehrere Erweiterungen, vorzugweise lamellenartige Erweiterungen, weiter vorzugsweise ein oder mehrere sich in eine Längsrichtung der Strahlenschutzpaneele erstreckende Erweiterungen auf, die dazu ausgebildet sind, einen zusätzlichen Strahlenschutz in einer Umgebung der Strahlenschutzpaneele bereitzustellen.

Die Erweiterung bzw. die Erweiterungen kann/können mit dem Strahlenschutzverbindungselement eine einteilige Einheit bilden oder als separate Erweiterung an dem Strahlenschutzverbindungselement lösbar befestigt sein bzw. von diesem abnehmbar sein, beispielsweise mittels Druckknopfverbindungen, Klettverbindungen, Karabinern, Haken und Ösen, Magneten und/oder anderen, zweckmäßigen Verbindungsmitteln.

Bevorzugt sind die ein oder mehreren Erweiterungen derart geformt, dass sie auf einem Patientenkörper ablegbar sind und/oder an einen Patientenkörper anpassbar, vorzugsweise ergonomisch anpassbar, sind und/oder ein Gewicht von maximal 3 kg, vorzugsweise maximal 1500 g, weiter vorzugsweise maximal 1000 g aufweisen.

Bevorzugt sind die Erweiterungen Strahlenschutz-Abdeckungen (Drapes). Die Drapes können an das Strahlenschutzverbindungselement anbringbar und/oder von dem Strahlenschutzverbindungselement abnehmbar sein.

Bevorzugt weisen die Strahlenschutzpaneele je ein oder mehrere Verbindungselemente zum Verbinden des jeweiligen Strahlenschutzpaneels mit einem jeweiligen Tragarm auf, wobei die ein oder mehreren Verbindungselemente ein Dreh- und Neigungsgelenk zum Drehen und Neigen des Paneels aufweisen können, und ist das Strahlenschutzverbindungselement dazu ausgebildet, nur an den Strahlenschutzpaneelen und nicht an den Tragarmen befestigt zu sein.

### Kurzbeschreibung der Figuren

- **Figur 1**: zeigt eine Ansicht einer Ausführungsform der erfindungsgemäßen Strahlenschutzvorrichtung.
- **Figur 2**: zeigt die Anordnung von **Figur 1** von einer Rückseite.
- **Figur 3**: zeigt eine Ausführungsform der erfindungsgemäßen Strahlenschutzvorrichtung, bei der die beiden Strahlenschutzpaneele teilweise überlappend angeordnet sind.
- **Figur 4**: zeigt eine Ausführungsform der erfindungsgemäßen Strahlenschutzvorrichtung, bei der die beiden Strahlenschutzpaneele in einem Winkel zueinander angeordnet sind.
- **Figur 5**: zeigt ein einzelnes Strahlenschutzpaneel der erfindungsgemäßen Strahlenschutzvorrichtung, das von dem Strahlenschutzverbindungselement gelöst ist.

### Detaillierte Beschreibung

Innerhalb der Figuren bezeichnen gleiche Bezugszeichen gleiche Gegenstände oder Komponenten.

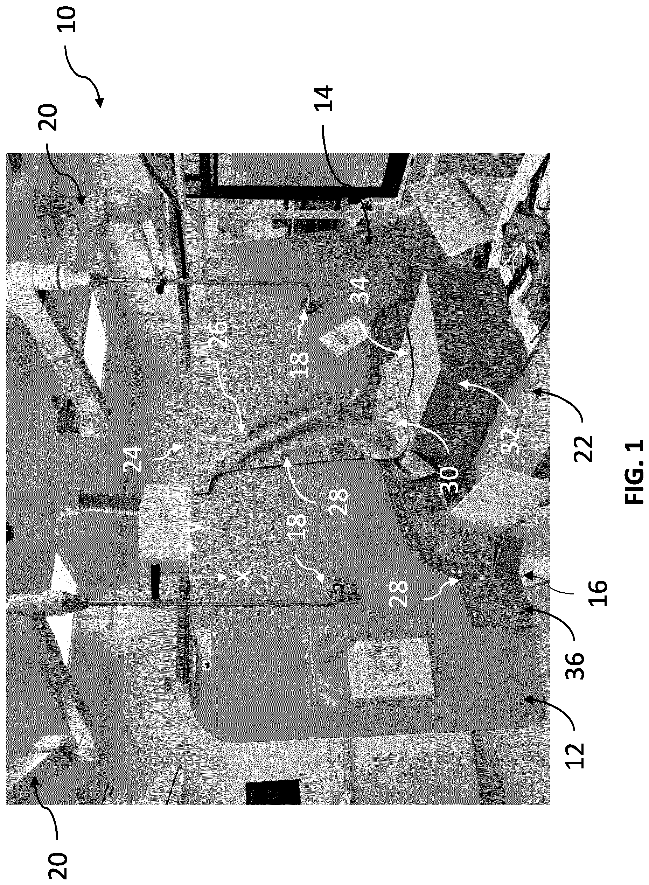

**Figur 1** zeigt eine Ansicht einer Ausführungsform einer erfindungsgemäßen Strahlenschutzvorrichtung 10.

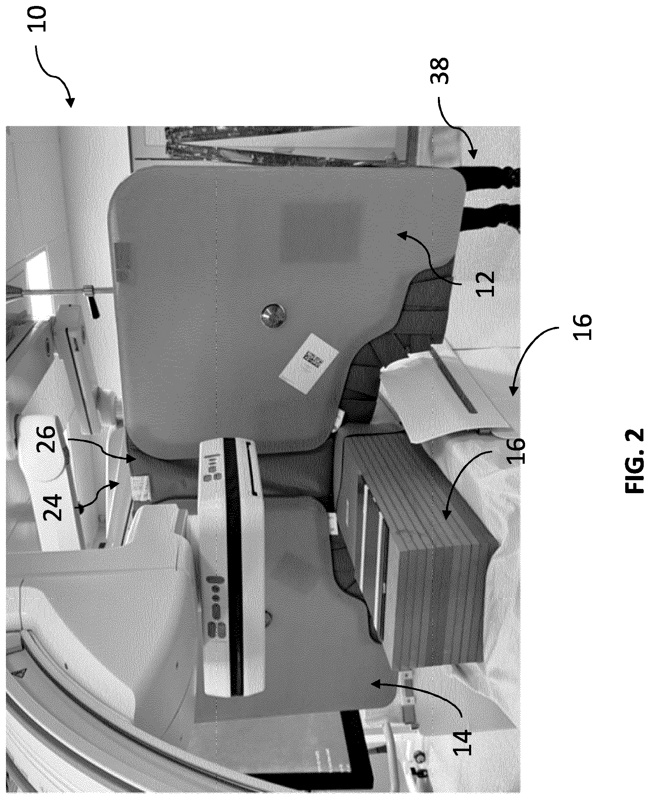

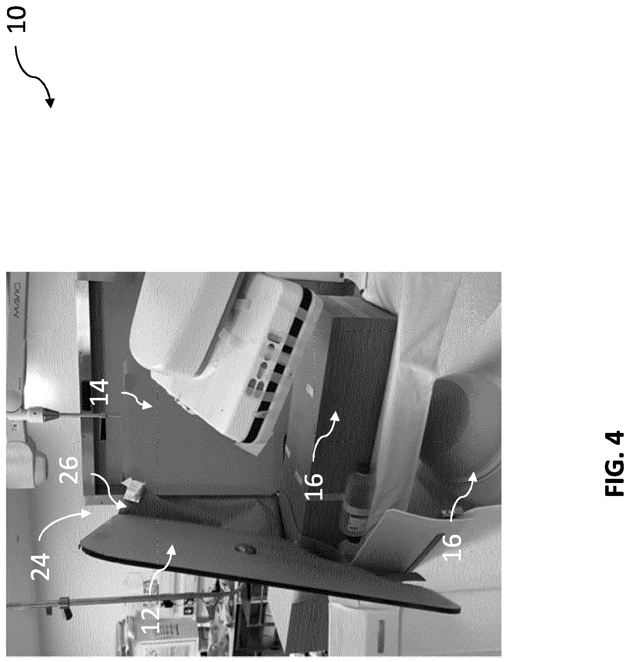

Die Strahlenschutzvorrichtung 10 weist ein erstes Strahlenschutzpaneel 12 und ein separates zweites Strahlenschutzpaneel 14 auf. Die Strahlenschutzpaneele 12, 14 können auch als Strahlenschutzplatten, Strahlenschutzscheiben, Strahlenschutzwände, oder Obertisch-Streustrahlenschutz bezeichnet werden. Sie können nicht transparent oder transparent ausgebildet sind. Die Paneele 12, 14 sind selbsttragend, d.h. relativ starr beziehungsweise steif und nicht flexibel beziehungsweise nicht biegsam. Die Paneele 12, 14 sind aus einem strahlenabsorbierenden Material mit einem Bleigleichwert Pb von 0,25 mm oder 0,5 mm oder 1,0 mm oder größer hergestellt, sodass eine Person vor schädlicher Strahlung, beispielsweise vor schädlicher Strahlung einer Strahlungsquelle 16, zumindest anteilig geschützt sind. Die Strahlungsquelle 16 kann eine Primärstrahlungsquelle, beispielsweise eine Röntgenröhre unter dem Tisch (angedeutet durch den Pfeil 16), und/oder eine Sekundärstrahlungsquelle, beispielsweise ein von Röntgenstrahlung durchstrahlter Patientenkörper sein. In **Figuren 2** **und** **4** sind die Primär- und Sekundärstrahlungsquellen 16 zu sehen.

Die Paneele 12, 14 weisen je ein Verbindungselement 18 auf, welches eine Befestigung und Verbindung eines jeweiligen Paneels 12, 14 an einem jeweiligen Tragarm 20 ermöglicht. Das Verbindungselement 18 kann ein Dreh- und Neigungsgelenk zum Drehen und Neigen der Paneele 12, 14 aufweisen. Das jeweilige Paneel 12, 14 kann mittels des jeweiligen Tragarms 20 um verschiedene Achsen gedreht und entlang verschiedener Achsen bewegt beziehungsweise verschoben werden. Die Paneele 12, 14 können so in beliebig zweckmäßigen Anordnungen zueinander angeordnet sein. Im konkreten Beispiel ist das erste Paneel 12 auf einer linken Seite eines Untersuchungstischs 22 angeordnet und ist das zweite Paneel 14 auf einer rechten Seite des Untersuchungstischs 22 angeordnet.

In anderen nicht gezeigten Ausführungsformen können die Paneele 12, 14 auch als mobile Strahlenschutzwände ausgebildet sein. Die mobilen Strahlenschutzwände können im Arbeitsbereich verfahren werden und sind nicht an einem Tragarm 20 befestigt.

Die beiden Paneele 12, 14 sind mit einem Spalt 24 zueinander angeordnet. Der Spalt 24 ist in **Figur 2** gut zu erkennen. Durch den Spalt 24 kann schädliche Strahlung hindurchtreten, beispielsweise Streustrahlung, sodass Personen einer durch den Spalt 24 tretenden Strahlung ausgesetzt sind.

Erfindungsgemäß wird dieser Spalt 24 durch ein Strahlenschutzverbindungselement 26 geschlossen. Das Strahlenschutzverbindungselement 26 ist derart flexibel ausgebildet, dass der Spalt 24 bei Bewegung der Paneele 12, 14 relativ zueinander und/oder bei unterschiedlicher Positionierung der Paneele 12, 14 untereinander geschlossen bleibt. Der Spalt 24 ist in Strahlungsrichtung durch das Strahlenschutzverbindungselement 26 geschlossen. Darunter soll verstanden werden, dass der Spalt 24 derart geschlossen ist, dass die in Strahlungsrichtung durch den Spalt hindurchtretende schädliche Strahlung reduziert werden kann. Die Strahlungsrichtung kann beliebig sind. Das Strahlenschutzverbindungselement 26 schließt den Spalt 24 in Strahlungsrichtung derart, dass eine in Strahlungsrichtung befindliche Person eine größtmögliche Reduktion der schädlichen Strahlung gemäß des zutreffenden Bleigleichwertes erfährt.

Das Strahlenschutzverbindungselement 26 ist aus einem flexiblen Material hergestellt. Als Material kann dasselbe oder ein vergleichbares Material gewählt werden, wie es beispielsweise auch bei Strahlenschutzkleidung und/oder bei Strahlenschutz-Drapes verwendet wird. Das Strahlenschutzverbindungselement 26 ist strahlungsabsorbierend. Das Strahlenschutzverbindungselement 26 kann einen Bleigleichwert Pb in einem Bereich von 0,25 mm bis 1,5 mm, vorzugweise in einem Bereich von 0,35 mm bis 0,5 mm, weiter vorzugsweise 0, 5mm oder 1,0 mm oder einen an den Bleigleichwert der Strahlenschutzpaneele angeglichenen Bleigleichwert aufweisen. Das Strahlenschutzverbindungselement 26 ist nicht selbstragend, ähnlich der Strahlenschutzkleidung. Mit anderen Worten würde das Strahlenschutzverbindungselement 26 ohne Befestigung an den Paneelen 12, 14 in sich zusammenfallen.

Das Strahlenschutzverbindungselement 26 ist lösbar an den Paneelen 12, 14 befestigt. Die Befestigung ist derart, dass das Strahlenschutzverbindungselement 26 ohne Werkzeugeinsatz an Befestigungselementen der Paneele 12, 14 befestigbar ist und von diesen zerstörungsfrei lösbar ist. Das Strahlenschutzverbindungselement 26 weist Gegenelemente auf, die in Eingriff mit den Befestigungselementen der Paneele 12, 14 bringbar sind und zerstörungsfrei von diesen gelöst werden können. Im konkreten Beispiel wird eine Druckknopfbefestigung 28 zwischen den Paneelen 12, 14 und dem Strahlenschutzverbindungselement 26 verwendet. Eine andere denkbare Befestigungsmöglichkeit wäre eine Klettverbindung. Weitere denkbare Befestigungsmöglichkeiten wären Karabiner, Haken und Ösen, Magnete, Saugnäpfe, Klebeverbindung.

In anderen, nicht gezeigten Ausführungsformen kann das Strahlenschutzverbindungselement 26 fest mit den Paneelen 12, 14 verbunden sein und nicht zerstörungsfrei und/oder nicht ohne Einsatz von (geeignetem) Werkzeug von den Paneelen 12, 14 entfernbar sein. Beispielsweise könnte es von einem Betreiber einer Röntgeneinrichtung und/oder von einem Strahlenschutzverantwortlichen explizit gewünscht sein, dass das Personal am Arbeitsplatz aus eigenem Antrieb bzw. mit eigenen Mitteln den erweiterten Strahlenschutz bzw. das Strahlenschutzverbindungselement 26 nicht entfernen können darf.

Das Strahlenschutzverbindungselement 26 kann als Einweg- oder als Mehrwegprodukt ausgebildet sein. Bei einem als Einwegprodukt ausgebildeten Strahlenschutzverbindungselement 26 kann das Strahlenschutzverbindungselement 26 mittels einer Klebeverbindung an den Paneelen 12, 14 angebracht sein. Bevorzugt kann das Strahlenschutzverbindungselement 26 durch Lösen der Klebeverbindung von den Paneelen 12, 14 gelöst werden, bevorzugt zerstörungsfrei und/oder ohne Einsatz von Werkzeug.

Das Strahlenschutzverbindungselement 26 kann reinigbar, und/oder sterilisierbar, und/oder desinfizierbar sein.

Das Strahlenschutzverbindungselement 26 kann eine Außenhülle (nicht gezeigt) aufweisen. In einem Inneren der Außenhülle kann ein geeignetes Strahlenschutzmaterial angeordnet sein. Alternativ oder zusätzlich kann ein geeignetes Strahlenschutzmaterial in die Außenhülle eingearbeitet ist. Die Außenhülle kann eine sterile Außenhülle (Umhüllung) sein. Die Außenhülle kann sterilisierbar und/oder desinfizierbar sein.

Im konkreten Beispiel weist das Strahlenschutzverbindungselement 26 eine Erweiterung 30 auf. Die Erweiterung 30 ist dazu ausgebildet, einen zusätzlichen Strahlenschutz in einer Umgebung der Strahlenschutzpaneele 12, 14 bereitzustellen, beispielsweise um weitere Spalte oder Lücken zwischen den Paneelen 12, 14 und dem Untersuchungstisch 22 und/oder dem Patientenkörper zu schließen und/oder Bereiche des Patientenkörpers, welche hinter den Paneelen 12, 14 anschließen, abzudecken.

Im konkreten Beispiel ist die Erweiterung 30 derart geformt, dass die Erweiterung 30 auf einem durch Bretter angedeuteten Patientenkörper 32 ablegbar ist. Die Erweiterung 30 kann so geformt sein, dass sie an den Patientenkörper 32 anpassbar, vorzugsweise ergonomisch anpassbar ist, ähnlich eines Strahlenschutz-Drapes 34, das in **Figur 1** ebenfalls gezeigt ist. Die Erweiterung 30 kann so geformt sein, dass ein Gewicht (beziehungsweise eine auf den Patientenkörper 32 wirkende Gewichtskraft) maximal 3 kg, vorzugsweise maximal 1,5 kg, weiter vorzugsweise maximal 1,0 kg ist.

Im konkreten Beispiel bildet die Erweiterung 30 mit dem Strahlenschutzverbindungselement 26 eine einteilige Einheit. In anderen nicht gezeigten Ausführungsformen kann die Erweiterung 30 als separate Erweiterung ausgebildet sein, die an dem Strahlenschutzverbindungselement 24 lösbar befestigt sein bzw. von diesem abnehmbar, vorzugsweise zerstörungsfrei und/oder ohne Werkzeugeinsatz abnehmbar, ist, beispielsweise mittels Druckknopfverbindungen, Klettverbindungen, Karabinern, Haken und Ösen, Magneten und/oder anderen, zweckmäßigen Verbindungsmitteln. Die Erweiterung 30 selbst kann eine Strahlenschutz-Abdeckung (Drape) sein, die an das Strahlenschutzverbindungselement 26 anbringbar und/oder von dem Strahlenschutzverbindungselement 26 abnehmbar, vorzugsweise zerstörungsfrei und/oder ohne Werkzeugeinsatz abnehmbar, ist.

Die Erweiterung 30 kann zumindest teilweise mit einem an den Paneelen 12, 14 befestigten Lamellenbehang 36 überlappen. Der Lamellenbehang 36 kann ebenfalls mittels einer Druckknopfverbindung 28 an den Paneelen 12, 14 befestigt sein und dient einem zusätzlichen Strahlenschutz, um beispielsweise Spalte zwischen den Paneelen 12, 14 und dem Untersuchungstisch 22 und/oder dem Patientenkörper zu schließen und/oder Bereiche des Patientenkörpers, welche hinter den Paneelen 12, 14 anschließen, abzudecken. In anderen nicht gezeigten Ausführungsbeispielen kann das Strahlenschutzverbindungselement 26 mehr als eine Erweiterung 30 aufweisen.

Im konkreten Beispiel von **Figur 1** weist das Strahlenschutzverbindungselement 26 eine im Wesentlichen rechteckige Form auf, wobei eine näherungsweise in x-Richtung gemessene Höhe beziehungsweise Länge des Strahlenschutzverbindungselements 26 größer ist als eine näherungsweise in y-Richtung gemessene Breite des Strahlenschutzverbindungselements 26. Die Höhe (beziehungsweise Länge) des Strahlenschutzverbindungselements 26 kann um einen Faktor in einem Bereich von 1 bis 20, vorzugweise in einem Bereich von 1 bis 10, weiter vorzugsweise in einem Bereich von 1 bis 5, größer als die Breite sein. Das Strahlenschutzverbindungselement 26 kann eine Höhe (beziehungsweise Länge) in einem Bereich von 40 cm bis 150 cm, vorzugsweise in einem Bereich von 50 cm bis 120 cm, weiter vorzugsweise in einem Bereich von 60 cm bis 100 cm aufweisen und/oder kann eine Höhe (beziehungsweise Länge) in einem Bereich einer Höhe der Strahlenschutzpaneele 12, 14 aufweisen und/oder kann eine Breite in einem Bereich von 10 cm bis 60 cm, vorzugsweise in einem Bereich von 20 cm bis 50 cm aufweisen.

Es sei nun auf **Figur 2** verwiesen, in der die Strahlenschutzvorrichtung 10 von **Figur 1** von einer Rückseite gezeigt ist. In der Ansicht von **Figur 2** befindet sich die Strahlungsquelle 16 vor den Paneelen 12, 14 und würde sich eine in Strahlungsrichtung befindliche Person 38 hinter den Paneelen 12, 14 befinden. Wie in Zusammenhang mit **Figur 1** erwähnt, kann die Strahlungsquelle eine Primärstrahlungsquelle, beispielsweise eine Röntgenröhre unter dem Tisch (angedeutet durch den Pfeil 16), wie auch eine Sekundärstrahlungsquelle, beispielsweise ein von Röntgenstrahlung durchstrahlter Patientenkörper (angedeutet durch einen weiteren Pfeil 16), sein. In **Figur 2** ist der erwähnte Spalt 24 zu sehen, der durch das Strahlenschutzverbindungselement 26 in Strahlungsrichtung geschlossen ist. Die hinter den Paneelen 12, 14 befindliche Person 38 ist durch den in Strahlungsrichtung geschlossenen Spalt 24 vor schädlicher Strahlung geschützt.

In **Figur 2** sind die Paneelen 12, 14 nebeneinander und näherungsweise in einer gemeinsamen Ebene angeordnet.

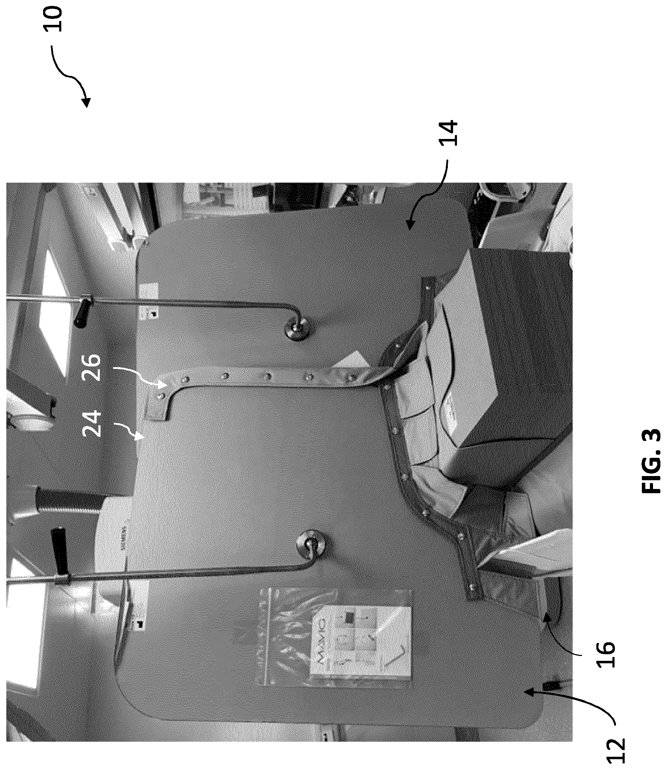

**Figur 3** zeigt eine Anordnung derselben Strahlenschutzvorrichtung 10, bei der die beiden Strahlenschutzpaneele 12, 14 teilweise überlappend angeordnet sind. In Beispiel von **Figur 3** befindet sich die Strahlungsquelle 16 hinter den Paneelen 12, 14 und befindet sich der Spalt 24 im Überlappungsbereich der beiden Paneele 12, 14. Wie in Zusammenhang mit **Figuren 1** **und** **2** erwähnt, kann die Strahlungsquelle eine Primärstrahlungsquelle, beispielsweise eine Röntgenröhre unter dem Tisch (angedeutet durch den Pfeil 16), wie auch eine Sekundärstrahlungsquelle, beispielsweise ein von Röntgenstrahlung durchstrahlter Patientenkörper sein. Das Strahlenschutzverbindungselement 26 erstreckt sich über den Überlappungsbereich und ist derart flexibel, dass der Spalt 24 auch bei teilweise überlappender Anordnung der Paneele 12, 14 geschlossen ist, sodass keine schädliche Strahlung durch den im Überlappungsbereich vorhandenen Spalt 24 hindurchtreten kann. Auch bei einem gewollten oder ungewollten Um positionieren/Verschieben der Paneele während der Prozedur, wobei beispielsweise der Überlappungsbereich aufgehoben wird, da die Paneele 12, 14 dann einen Abstand zueinander aufweisen, schließt das Strahlenschutzverbindungselement 26 die Lücke bzw. den Spalt 24. Das Strahlenschutzverbindungselement 26 ist ferner derart flexibel, dass das Strahlenschutzverbindungselement 26 um eine Kante des ersten und zweiten Paneels 12, 14 biegbar ist. Eine Breite des Strahlenschutzverbindungselements 26 kann derart gewählt sein, dass ein gewünschter Überlappungsbereich der Strahlenschutzpaneele 12, 14 durch das Strahlenschutzverbindungselement 26 abdeckbar ist.

**Figur 4** zeigt eine Stellung der Strahlenschutzvorrichtung 10, bei der die beiden Strahlenschutzpaneele 12, 14 in einem Winkel zueinander angeordnet sind. Der Winkel ist näherungsweise 90°, kann jedoch beliebig sein. Wie zu erkennen ist, ist das Strahlenschutzverbindungselement 26 derart flexibel, dass bei einer gewinkelten Anordnung der beiden Paneele 12, 14 der Spalt 24 durch das Strahlenschutzverbindungselement 26 geschlossen ist. In **Figur 4** ist zudem die unterhalb des Tisches angeordnete Röntgenröhre (Beispiel für eine (Primär)Strahlungsquelle 16) und der auf dem Tisch durch Bretter angeordnete Patientenkörper (Beispiel für eine (Sekundär)Strahlungsquelle 16) zu sehen.

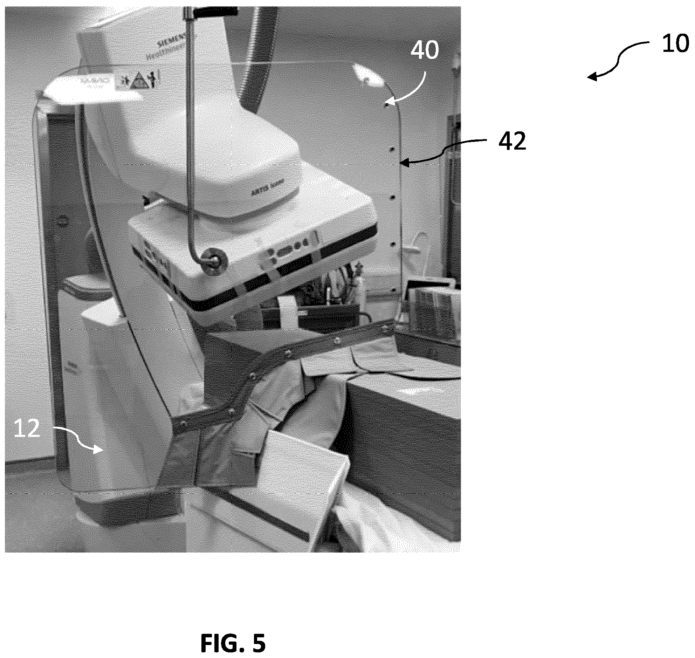

**Figur 5** zeigt das erste Strahlenschutzpaneel 12 ohne ein daran befestigtes Strahlenschutzverbindungselement. In **Figur 5** ist das Strahlenschutzpaneel 12 transparent ausgebildet. **Figur 5** zeigt die Befestigungselemente 40 der Druckknopfverbindung 28 (siehe beispielsweise **Figur 1****).** Die Gegenelemente (nicht gezeigt) sind am Strahlenschutzverbindungselement angebracht. Wie zu erkennen ist, sind die Befestigungselemente 40 in einem Randbereich des Paneels 12 angeordnet. Die Befestigungselemente 40 sind von einer Außenkante 42 des Paneels 12 derart beabstandet, dass das im Randbereich befestigte Strahlenschutzverbindungselement zumindest teilweise mit dem Paneel 12 überlappt. Das teilweise Überlappen von Paneel 12 und Strahlenschutzverbindungselement 26 im Randbereich des Paneels 12 ist beispielsweise in **Figur 3** zu sehen.

In **Figuren 1** bis **5** ist das Strahlenschutzverbindungselement 26 in Zusammenhang mit zwei Strahlenschutzscheiben (Beispiele für Paneele 12, 14) beschrieben worden. In anderen nicht gezeigten Ausführungsformen kann das Strahlenschutzverbindungselement 26 an einer Strahlenschutzscheibe (Beispiel für Paneele 12, 14) und einer (mobilen) Strahlenschutzwand (anderes Beispiel für Paneele 12, 14) oder in Kombination mit einer Strahlenschutzscheibe und einem am Tisch befestigten Unterkörperschutz verwendet werden. Der Unterkörperschutz kann ebenfalls ein Beispiel für ein Paneel 12, 14 sein.

## Patentansprüche

1. Strahlenschutzvorrichtung (10) aufweisend:
- ein erstes Strahlenschutzpaneel (12),
- ein von dem ersten Strahlenschutzpaneel (12) separat angeordnetes zweites Strahlenschutzpaneel (14), wobei die beiden Strahlenschutzpaneele (12, 14) derart zueinander angeordnet sind, dass zwischen den beiden Strahlenschutzpaneelen (12, 14) ein für Strahlung durchlässiger Spalt (24) vorhanden ist, und
- ein an dem ersten Strahlenschutzpaneel (12) und an dem zweiten Strahlschutzpaneel (14) befestigbares und den Spalt (24) schließendes Strahlenschutzverbindungselement (26), wobei das Strahlenschutzverbindungselement (26) derart flexibel ausgebildet ist, dass der Spalt (24) bei Bewegung und/oder bei einer unterschiedlichen Positionierung der Strahlenschutzpaneele (12, 14) geschlossen bleibt.

2. Strahlenschutzvorrichtung (10) nach Anspruch 1, wobei
das Strahlenschutzverbindungselement (26) nicht selbsttragend ist, und/oder das Strahlenschutzverbindungselement (26) derart flexibel ist, dass das Strahlenschutzverbindungselement (26) um eine Kante des ersten und/oder zweiten Strahlenschutzpaneels (12, 14) biegbar ist.

3. Strahlenschutzvorrichtung (10) nach einem der vorhergehenden Ansprüche, wobei die Strahlenschutzpaneele (12, 14) in verschiedenen Winkeln zueinander positionierbar sind und das Strahlenschutzverbindungselement (26) dazu ausgebildet ist, den Spalt (24) in gewinkelten Anordnungen der Strahlenschutzpaneele (12, 14) zu schließen.

4. Strahlenschutzvorrichtung (10) nach einem der vorhergehenden Ansprüche, wobei die Strahlenschutzpaneele (12, 14) teilweise überlappend zueinander positionierbar sind und das Strahlenschutzverbindungselement (26) dazu ausgebildet ist, den Spalt (24) in teilweise überlappenden Anordnungen der Strahlenschutzpaneele (12, 14) zu schließen, vorzugweise wobei eine Breite des Strahlenschutzverbindungselement (26) derart gewählt ist, dass ein gewünschter Überlappungsbereich der Strahlenschutzpaneele (12, 14) durch das Strahlenschutzverbindungselement (26) abdeckbar ist.

5. Strahlenschutzvorrichtung (10) nach einem der vorhergehenden Ansprüche, wobei das erste und zweite Strahlenschutzpaneel (12, 14) Befestigungselemente (40) zum lösbaren Befestigen des Strahlenschutzverbindungselements (26) aufweisen.

6. Strahlenschutzvorrichtung (10) nach Anspruch 6, wobei
die Befestigungselemente (40) in einem Randbereich des jeweiligen Strahlenschutzpaneels (12, 14) angeordnet sind, vorzugweise wobei die Befestigungselement (40) von einer Außenkante (42) des jeweiligen Strahlenschutzpaneels (12, 14) derart beabstandet angeordnet sind, dass das im Randbereich befestigte Strahlenschutzverbindungselement (26) zumindest teilweise mit dem jeweiligen Strahlenschutzpaneel (12, 14) überlappt, und/oder
das Strahlenschutzverbindungselement (26) Gegenelemente aufweist, die an den Befestigungselementen (40) der Strahlenschutzpaneele (12, 14) befestigbar sind, vorzugsweise wobei die Gegenelemente von den Befestigungselementen (40) zerstörungsfrei und ohne Werkzeugeinsatz lösbar sind.

7. Strahlenschutzvorrichtung (10) nach einem der vorhergehenden Ansprüche, wobei das Strahlenschutzverbindungselement (26) aus einem für Strahlenschutzkleidung geeignetem Material hergestellt ist, und/oder aus einem Strahlenschutz-Drape-Material hergestellt ist, und/oder ein Strahlenschutz-Drape ist.

8. Strahlenschutzvorrichtung (10) nach einem der vorhergehenden Ansprüche, wobei das Strahlenschutzverbindungselement (26) einen Bleigleichwert Pb in einem Bereich von 0,25 mm bis 1,5 mm, vorzugsweise in einem Bereich von 0,35 mm bis 0, 5 mm, weiter vorzugsweise 0,5 mm bis 1,0 mm oder einen auf den Bleigleichwert der Strahlenschutzpaneele angeglichenen Bleigleichwert, aufweist.

9. Strahlenschutzvorrichtung (10) nach einem der vorhergehenden Ansprüche, wobei das Strahlenschutzverbindungselement (26)
- ein Gewicht in einem Bereich von 0,3 kg bis 10 kg, vorzugsweise in einem Bereich von 1 kg bis 6 kg, weiter vorzugsweise in einem Bereich von 1 kg bis 4 kg, aufweist, und/oder
- ein Gewicht von maximal 10 kg, vorzugsweise maximal 5 kg, weiter vorzugsweise maximal 4 kg aufweist, und/oder
- eine Höhe in einem Bereich von 40 cm bis 150 cm, vorzugsweise in einem Bereich von 50 cm bis 120 cm, weiter vorzugsweise in einem Bereich von 60 cm bis 100 cm aufweist, und/oder
- eine Höhe in einem Bereich einer Höhe der Strahlenschutzpaneele (12, 14) aufweist, und/oder
- eine Breite in einem Bereich von 10 cm bis 60 cm, vorzugsweise in einem Bereich von 20 cm bis 50 cm aufweist, und/oder
- eine im Wesentlichen rechteckige Form aufweist, wobei eine Höhe des Strahlenschutzverbindungselements (26) größer als eine Breite des Strahlenschutzverbindungselements (26) ist, vorzugsweise wobei die Höhe um einen Faktor in einem Bereich von 1 bis 20, vorzugweise in einem Bereich von 1 bis 10, weiter vorzugsweise in einem Bereich von 1 bis 5, größer als die Breite ist.

10. Strahlenschutzvorrichtung (10) nach einem der vorhergehenden Ansprüche, wobei das Strahlenschutzverbindungselement (26) ein oder mehrere Erweiterungen (30), vorzugweise lamellenartige Erweiterungen, weiter vorzugsweise ein oder mehrere sich in eine Längsrichtung der Strahlenschutzpaneele (12, 14) erstreckende Erweiterungen (30) aufweist, die dazu ausgebildet sind, einen zusätzlichen Strahlenschutz in einer Umgebung der Strahlenschutzpaneele (12, 14) bereitzustellen.

11. Strahlenschutzvorrichtung (10) nach Anspruch 10, wobei die ein oder mehreren Erweiterungen (30) derart geformt sind, dass
- sie auf einem Patientenkörper (32) ablegbar sind und/oder
- an einen Patientenkörper (32) anpassbar, vorzugsweise ergonomisch anpassbar, sind und/oder
- ein Gewicht von maximal 3 kg, vorzugsweise maximal 1,5 kg, weiter vorzugsweise maximal 1 kg aufweisen, und/oder
- die ein oder mehreren Erweiterungen (30) abnehmbar sind, und/oder ein oder mehrere Strahlenschutz-Drapes sind.

12. Strahlenschutzvorrichtung (10) nach einem der vorhergehenden Ansprüche, wobei die Strahlenschutzpaneele (12, 14) ein Verbindungselement zum Verbinden des jeweiligen Strahlenschutzpaneels (12, 14) mit einem jeweiligen Tragarm (20) aufweisen und das Strahlenschutzverbindungselement (26) dazu ausgebildet ist nur an den Strahlenschutzpaneelen (12, 14) und nicht an den Tragarmen (20) befestigt zu sein.

13. Strahlenschutzvorrichtung (10) nach einem der vorhergehenden Ansprüche, wobei das Strahlenschutzverbindungselement (26) als Einwegprodukt ausgebildet ist und mittels einer Klebeverbindung an die Strahlenschutzpaneele (12, 14) anbringbar und vorzugsweise zerstörungsfrei und ohne Werkzeugeinsatz von den Strahlenschutzpaneelen (12, 14) lösbar ist.

14. Strahlenschutzvorrichtung (10) nach einem der vorhergehenden Ansprüche, wobei das Strahlenschutzverbindungselement (26) eine Außenhülle aufweist und in einem inneren Bereich der Außenhülle ein Strahlenschutzmaterial angeordnet ist.

15. Strahlenschutzvorrichtung (10) nach Anspruch 14, wobei die Außenhülle eine sterile Umhüllung ist, und/oder sterilisierbar ist, und/oder desinfizierbar ist.

## Geänderte Patentansprüche

### Geänderte Patentansprüche gemäss Regel 137(2) EPÜ.

1. Strahlenschutzvorrichtung (10) aufweisend:
- ein erstes Strahlenschutzpaneel (12),
- ein von dem ersten Strahlenschutzpaneel (12) separat angeordnetes zweites Strahlenschutzpaneel (14), wobei die beiden Strahlenschutzpaneele (12, 14) derart zueinander angeordnet sind, dass zwischen den beiden Strahlenschutzpaneelen (12, 14) ein für Strahlung durchlässiger Spalt (24) vorhanden ist, und
- ein an dem ersten Strahlenschutzpaneel (12) und an dem zweiten Strahlschutzpaneel (14) befestigbares und den Spalt (24) schließendes Strahlenschutzverbindungselement (26), wobei das Strahlenschutzverbindungselement (26) derart flexibel ausgebildet ist, dass der Spalt (24) bei Bewegung und/oder bei einer unterschiedlichen Positionierung der Strahlenschutzpaneele (12, 14) geschlossen bleibt, wobei
- die Strahlenschutzpaneele (12, 14) in verschiedenen Winkeln zueinander positionierbar sind und das Strahlenschutzverbindungselement (26) dazu ausgebildet ist, den Spalt (24) in gewinkelten Anordnungen der Strahlenschutzpaneele (12, 14) zu schließen, und
- die Strahlenschutzpaneele (12, 14) ein Verbindungselement zum Verbinden des jeweiligen Strahlenschutzpaneels (12, 14) mit einem jeweiligen Tragarm (20) aufweisen und das Strahlenschutzverbindungselement (26) dazu ausgebildet ist nur an den Strahlenschutzpaneelen (12, 14) und nicht an den Tragarmen (20) befestigt zu sein.

2. Strahlenschutzvorrichtung (10) nach Anspruch 1, wobei
das Strahlenschutzverbindungselement (26) nicht selbsttragend ist.

3. Strahlenschutzvorrichtung (10) nach einem der vorhergehenden Ansprüche, wobei die Strahlenschutzpaneele (12, 14) teilweise überlappend zueinander positionierbar sind und das Strahlenschutzverbindungselement (26) dazu ausgebildet ist, den Spalt (24) in teilweise überlappenden Anordnungen der Strahlenschutzpaneele (12, 14) zu schließen, vorzugweise wobei eine Breite des Strahlenschutzverbindungselement (26) derart gewählt ist, dass ein gewünschter Überlappungsbereich der Strahlenschutzpaneele (12, 14) durch das Strahlenschutzverbindungselement (26) abdeckbar ist.

4. Strahlenschutzvorrichtung (10) nach einem der vorhergehenden Ansprüche, wobei das erste und zweite Strahlenschutzpaneel (12, 14) Befestigungselemente (40) zum lösbaren Befestigen des Strahlenschutzverbindungselements (26) aufweisen.

5. Strahlenschutzvorrichtung (10) nach Anspruch 4, wobei
die Befestigungselemente (40) in einem Randbereich des jeweiligen Strahlenschutzpaneels (12, 14) angeordnet sind, vorzugweise wobei die Befestigungselement (40) von einer Außenkante (42) des jeweiligen Strahlenschutzpaneels (12, 14) derart beabstandet angeordnet sind, dass das im Randbereich befestigte Strahlenschutzverbindungselement (26) zumindest teilweise mit dem jeweiligen Strahlenschutzpaneel (12, 14) überlappt.

6. Strahlenschutzvorrichtung (10) nach Anspruch 5, wobei das Strahlenschutzverbindungselement (26) Gegenelemente aufweist, die an den Befestigungselementen (40) der Strahlenschutzpaneele (12, 14) befestigbar sind, vorzugsweise wobei die Gegenelemente von den Befestigungselementen (40) zerstörungsfrei und ohne Werkzeugeinsatz lösbar sind.

7. Strahlenschutzvorrichtung (10) nach einem der vorhergehenden Ansprüche, wobei das Strahlenschutzverbindungselement (26) aus einem für Strahlenschutzkleidung geeignetem Material hergestellt ist.

8. Strahlenschutzvorrichtung (10) nach einem der vorhergehenden Ansprüche, wobei das Strahlenschutzverbindungselement (26) einen Bleigleichwert Pb in einem Bereich von 0,25 mm bis 1,5 mm, vorzugsweise in einem Bereich von 0,35 mm bis 0, 5 mm, weiter vorzugsweise 0,5 mm bis 1,0 mm oder einen auf den Bleigleichwert der Strahlenschutzpaneele angeglichenen Bleigleichwert, aufweist.

9. Strahlenschutzvorrichtung (10) nach einem der vorhergehenden Ansprüche, wobei das Strahlenschutzverbindungselement (26) eine im Wesentlichen rechteckige Form aufweist, wobei eine Höhe des Strahlenschutzverbindungselements (26) größer als eine Breite des Strahlenschutzverbindungselements (26) ist, vorzugsweise wobei die Höhe um einen Faktor in einem Bereich von 1 bis 20, vorzugweise in einem Bereich von 1 bis 10, weiter vorzugsweise in einem Bereich von 1 bis 5, größer als die Breite ist.

10. Strahlenschutzvorrichtung (10) nach einem der vorhergehenden Ansprüche, wobei das Strahlenschutzverbindungselement (26) ein oder mehrere Erweiterungen (30), vorzugweise lamellenartige Erweiterungen, weiter vorzugsweise ein oder mehrere sich in eine Längsrichtung der Strahlenschutzpaneele (12, 14) erstreckende Erweiterungen (30) aufweist, die dazu ausgebildet sind, einen zusätzlichen Strahlenschutz in einer Umgebung der Strahlenschutzpaneele (12, 14) bereitzustellen.

11. Strahlenschutzvorrichtung (10) nach Anspruch 10, wobei die ein oder mehreren Erweiterungen (30) abnehmbar und derart geformt sind, dass auf einem Patientenkörper (32) ablegbar sind.

12. Strahlenschutzvorrichtung (10) nach einem der vorhergehenden Ansprüche, wobei das Strahlenschutzverbindungselement (26) derart flexibel ist, dass das Strahlenschutzverbindungselement (26) um eine Kante des ersten und/oder zweiten Strahlenschutzpaneels (12, 14) biegbar ist.

13. Strahlenschutzvorrichtung (10) nach einem der vorhergehenden Ansprüche, wobei das Strahlenschutzverbindungselement (26) als Einwegprodukt ausgebildet ist und mittels einer Klebeverbindung an die Strahlenschutzpaneele (12, 14) anbringbar und vorzugsweise zerstörungsfrei und ohne Werkzeugeinsatz von den Strahlenschutzpaneelen (12, 14) lösbar ist.

14. Strahlenschutzvorrichtung (10) nach einem der vorhergehenden Ansprüche, wobei das Strahlenschutzverbindungselement (26) eine Außenhülle aufweist und in einem inneren Bereich der Außenhülle ein Strahlenschutzmaterial angeordnet ist.

15. Strahlenschutzvorrichtung (10) nach Anspruch 14, wobei die Außenhülle sterilisierbar und/oder desinfizierbar ist.
